# NEW EUROPEAN PATENT SPECIFICATION

(11) **EP 1 557 179 B2**
(45) Date of publication and mention of the opposition decision: **31.08.2011**
(45) Mention of the grant of the patent: 16.01.2008
(21) Application number: 05100373.9
(22) Date of filing: 21.01.2005
(51) Int. Cl.: A61K 47/36

(54) **Analgesic dosage forms that are unable to be inhaled or injected**
Analgistische Darreichungsform, die nicht parenteral oder inhalation dosiert werden können
Forme posologuque analgesique ne pouvant pas être inhalé ou injecté

(30) Priority: 22.01.2004 US 762714
(43) Date of publication of application: 27.07.2005
(73) Proprietor: MAXWELL, Gordon, New York, NY 10028 (US)
(72) Inventor: MAXWELL, Gordon, New York, NY 10028 (US)
(74) Representative: Coletti, Raimondo

(56) References cited:
- WO-A-2004/037259
- WO-A1-95/20947
- DE-A1- 10 250 083
- US-A- 3 773 955
- US-A- 4 070 494
- US-A1- 2003 068 371
- RUBEN ET AL: "Temazepam misuse in a group of injecting drug users" BR.J.ADDICTION, vol. 87, 1992, pages 1387-1392, XP009044178
- ENNO FREYE: 'Opioide in der Medizin - 6. aktualisierte und erweiterte Auflage', 2004, SPRINGER VERLAG pages 95 - 98
- 'Remington: The Science and Practice of Pharmacy', vol. 20TH ED., 1995 pages 897 - 898

## Description

### FIELD OF INVENTION

The invention provides a means for reducing the potential for the abuse of potent opiate oral analgetic drugs by preventing the recovery of the opiate oral analgetic in a form that allows the preparation of a parenteral or inhalable dosage formulation.
This invention relates to solid dosage forms of oral analgetic drugs which are effective for pain control(or treating diarrhea) and are not adapted for recovery of the opiate analgetic. The invention also provides a novel process for preparing the novel formulations of the invention and reducing the side effects of analgetic preparations.

### BACKGROUND OF THE INVENTION

The term opiate applies to a legal classification of drugs that include those which are derived from Papaver somniferum and other drugs that have been listed by authorities as having the same or similar addictive potential or properties that were the basis for the regulation or prohibition of the use of derivatives of Papaver somniferum . Morphine and codeine are well known opiates that have previously been widely abused and in recent years the use of other derivatives of Papaver somniferum such as oxycodone have been widely abused because it is not difficult to prepare an injectable form of oxycodone by merely dissolving the oral oxycontin tablets in water and thus preparing an injectable form of the oxycodone. U.S. 3,773,955 describes the making of a composition of oxycodone and naloxone to prevent the abuse of oxycodone by taking advantage of the known opiate blocking effect of naloxone. Combinations of pentazocine and naloxone and burenorphine and naloxone have also been described. However, these formulations, which contain naloxone, have been relatively easy to separate using high performance liquid chromatography (HPLC)or other techniques.

In the prior art, paregoric (camphorated tincture of opium) was sold to the general public as a remedy for diarrhea or for teething pain because it contained a small amount of opium. The sale of this preparation without a prescription was discontinued because addicted individuals would separate the camphor by cooling and/or filtering the preparation, boiling off the alcohol and then re-dissolving the opium containing residue in water to make an injectable preparation. This resulted in the loss to the general public of an effective diarrhea remedy which is more effective and faster acting than the insoluble drugs Lomotil and Imodium which are widely used. Furthermore, Lomotil and Immodium are toxic to children thus the FDA bans the use of these drugs in this patient population.
Methadone is an opiate analgetic that has been available in tablet and liquid formulations for more than 50 years. This drug is an important drug in the treatment of opiate addiction in many dependent individuals. Typically, methadone is either administered at a clinic to an addicted patient as an oral liquid in the presence of a health professional to reduce the potential for diversion of the drug for street abuse by addicted persons who are not under treatment and typically use methadone in combination with other drugs. A supply of the liquid methadone is usually provided for self-administration by the patient use between clinic visits typically in the form of a Kool_Aid or other liquid flavored solution. These solutions require refrigeration and accidental poisonings of children and other non-addicted individuals has been known to occur.

It is apparent that a need exists for oral dosage forms of opiate anagetics that are stable, contain an opiate antagonist, and are resistant to conventional separation techniques that are designed to permit recovery of the opiate in a form that is pure enough to prepare a parenteral dose of the drug for illicit use.

### SUMMARY OF THE INVENTION

The present invention is based on the discovery that an opiate and an opiate antagonist may be combined in an oral dosage form with a hydrocolloid containing excipient that comprises a gel forming agent which swells in the presence of water and forms a gel type matrix that substantially prevents the selective extraction of the opiate from the opiate-opiate antagonist mixture or the use of the highly viscous hydrocolloid solution as a injectable preparation and provide a formulation having reduced side effects. The invention also includes solid oral dosage formulations of an opiate and a hydrocolloid excipient that comprises a gel forming agent which swells in the presence of water and forms a gel type matrix that substantially prevents the making of a parenteral injection of the opiate through the formation of the highly viscous matrix that can not be passed through a hypodermic needle.

Accordingly, it is a primary object of the present invention to provide a novel stable, oral dosage form of a combination of an orally effective opiate drug that cannot be made into a parenteral formulation of the opiate drug.

It is also an object of this invention to provide a solid dosage form of an opiate drug that forms a non-injectable, highly viscous gel when the solid dosage form is placed in water.

It is also an object of the invention to provide a method of formulating a solid oral dosage from an opiate and an opiate antagonist which is resistant to conventional separation techniques that may be applied to separate the opiate from the opiate antagonist by adding a hydrocolloid forming material to the solid dosage formulation.

These and other objects of the invention will become apparent from a review of the appended specification.

### DETAILED DESCRIPTION OF THE INVENTION

The above objects are realized by a solid oral dosage form, which comprises an opiate and an opiate antagonist, an excipient which comprises a hydrocolloid and which includes an amount of enteric coated opiate antagonist pellets, which is effective to prevent opiate induced constipation. The preferred form of the solid dosage form is a tablet but it is also possible to formulate the solid dosage form of the invention in hard or soft gelatin capsules. Without being bound by any theory under which the invention operates, it is believed that the addition of a hydrocolloid causes the solid dosage form to swell in the presence of water and form a highly viscous matrix or slurry that is impossible to pass through a hypodermic needle or pass through any known type of filtration means. The matrix that is formed also causes the soluble opiate and opiate antagonist to become trapped in the expanding matrix that the hydrocolloid forms as it is exposed to water and makes it difficult to use conventional separation techniques to obtain concentrated form of the opiate drug apart from the opiate antagonist and the hydrocolloid material.

The principal side effect that is avoided by the invention is constipation. This is achieved by the action of the separate opiate antagonist in enteric form and it is believed that the hydrocolloid also exerts a positive beneficial effect. The opiates that may be used in the invention include all known opiates including but not limited to morphine, codeine, dilaudid, pantopon, methadone, paregoric, pentazocine, buprenorphine, fentanyl, oxycodone, oxymorphone, hydromorphone, hydrocodone, propoxyphene , nalbuphine, meperidine and the like.

The solid pharmaceutical dosage forms of the invention include an amount of enteric coated opiate antagonist pellets which are effective to prevent opiate induced constipation. The amount, per unit dose of opiate, of the enteric coated opiate antagonist pellets may vary from 3 to 10mg per unit dose. The enteric coating agents include Eudragit S100, hydroxypropyl methylcellulose, polyvinylpyrrolidone, and the like. It is understood that where polymeric materials are used, the molecular weight will be selected to provide the desired effect.

The opiate antagonists include but are not limited to naloxone, naltrexone, methylnaltrexone, or naloxonazine. The preferred opiate antagonist is naloxone which has a very high oral/parenteral ratio, is completely devoid of agonist activity and is ideally suited for use as a denaturant for solid dosage forms of opiates.

The hydrocolloids that form a gel like matrix when contacted with water are well known and are described in the literature. These materials are generally defined as materials that include increase viscosity, and contribute to the thickening and/or gelation when contacted with water. The hydrocolloids include cellulose derivatives such as high viscosity hydroxypropyl methyl cellulose having a viscosity of above 3000 mPa s (2% aq. soln. @ 20°C), agar, alginates, zein from Zea mays (Zein F-4000)such as carrageenan, guar gum, locust bean gum, xanthan gum and the like.
As indicated above the dosage forms of the present invention may comprise auxiliary excipients such as for example diluents, binders, lubricants, surfactants, disintegrants, plasticisers, anti-tack agents, opacifying agents, pigments, and the like. As will be appreciated by those skilled in the art, the exact choice of excipient and their relative amounts will depend to some extent on the final oral dosage form.

Suitable diluents include for example pharmaceutically acceptable inert fillers such as microcrystalline cellulose, lactose, starch, dibasic calcium phosphate, saccharides, and/or mixtures of the foregoing. Examples of microcrystalline celluloses include (Avicel PH 200, Avicel PH 102, Avicel PH 112, Avicel PH 101, Avicel PH 3020; examples of lactose include lactose monohydrate, lactose; in additon, mannitol; sucrose; and dextrose may be used.

Suitable binders include for example starch, povidone, low viscosity hydroxypropylmethylcellulose such as Methocel E-5 Prem. LV, pregelatinised starch, hydroxypropylcellulose and/or mixtures of the foregoing.
Suitable lubricants, including agents that act on the flowability of the powder to be compressed are, for example, stearic acid, talc, colloidal slilcon dioxide, calcium or magnesium stearate, or sodium stearyl fumarate,

Suitable disintegrants include for example crosslinked polyvinyl pyrrolidone, various starches such as potato starch, corn starch, potato starch,rice starch and modified starches, crospovidone, sodium starch glycollate croscarmellose sodium, and the like or mixtures thereof. As indicated above the dosage forms of the present invention may comprise auxiliary, excipients such as for example lubricants, plasticisers, anti-tack agents, opacifying agents, ) pigments, and such like, As will be appreciated by those skilled in the art, the exact choice of excipient and their relative amounts will depend to some extent on the final oral dosage form.

Suitable lubricants, including agents that act on the flowability of the powder to be compressed are, for example, colloidal slilcon dioxide such as Aerosil 200 (Aerosil is a Trade Mark); talc; stearic acid, magnesium stearate, calcium stearate and sodium stearyl fumarate.

Granulations for preparing tablets according to the invention can be manufactured in accordance with standard procedures in which the opiate drug, the opiate antagonist and the hydrogel forming material may be combined with suitable excipients pr4ior to mixing and forming compressible granules by adding solution of a binder in a low or high shear mixer or by fluidized bed granulation. The granulate is dried, preferably in a fluidized bed dryer. The dried granulate is sieved and mixed with lubricants and disintegrants. Alternatively the manufacture of granules of can be achieved by direct mixing of the directly compressible excipients or by roller compaction.

The dosage forms of the invention will comprise a therapeutically effective amount of the opiate analgetic, an amount of the opiate antagonist which is effective to antagonize the additive potential of the opiate analgetic drug, an amount of the hydrocolloid which will cause the dosage form to be converted into a non-injectable gel like mass when the dosage form is placed in from 30 to 100ml of an aqueous fluid such as water, and an amount of enteric coated opiate antagonist pellets which is effective to prevent opiate induced constipation.

### EXAMPLE 1

### (Oxycodone-naloxone) (5 + 0.25) For analgesia

| Components | |
|---|---|
| Oxycodone hydrochloride | 500 gm |
| Naloxone hydrochloride | 40 gm |
| Starch U.S.P. (for paste) | 1000 gm |
| Starch U.S.P. (for granulation) | 40000 gm |
| Keltrol F (xanthan gum from Xanthamonas campetris) 950 gm (C.P. Kelco U.S., Wilmington, DE 19894) | |
| Locust bean gum from Seratonia siliqua (Degussa Texturant | 3700 gm |
| Systems U.S. Atlanta, GA 30340) Monobasic calcium phosphate | 700 gm |
| Dibasic calcium phosphate | 700 gm |
| Microcrystalline cellulose (Avicel) Biopolymers, Newark, DE) | 24800 gm (FMC |
| Kelcoloid HVF 18 (30 mesh propylene glycol alginate | 10000 gm (ISP |
| Alginates, San Diego CA 92113) | |
| F .D. and C. yellow lake no. 5 | 500 gm |
| Zein F-300 20-30 mesh from Zea mays (Freeman Industries LLC, Tuckahoe, NY 10701 | 5000 gm |
| Magnesium stearate U.S.P. | 950 gm |
| Total | 91225 gm |

A starch paste is prepared by mixing 1000 gm of starch with 8000 gm of deionized water. A separate blend is prepared by mixing 2500 gm of starch, 2000 gm of oxycodone hydrochloride and 400025 gm of anhydrous lactose. Naloxone hydrochloride (200 gm) is dissolved in 1500 gm of deionized water. The starch paste and the solution of naloxone hydrochloride are wet granulated with the dry blend of starch, methadone hydrochloride and anhydrous lactose. The wet granulation is passed through a No.10 mesh screen, spread on trays and dried for 18 hours at 120° F. The moisture content of the dried granulation is between 2.0-3.5%. The dried granulation is then consecutively passed through a No. 12 mesh screen and a No. 30 mesh screen.

To the dried granulation there are added 950 gm of xanthan gum (Keltrol F) (from Xanathamonas campestris), 3700 gm of locust bean gum (from Seratonia siliqua), 100 gm of monobasic calcium phosphate, 100 gm of dibasic calcium phosphate, 24800 gm of microcrystalline cellulose (Avicel), and 500 gm of F D & C yellow lake No.5 and the mixture is blended for 15 minutes. Propylene glycol alginate (Kelcoloid HVF) which has been compacted and granulated to produce 18-30 mesh granules 10000 gm), Zein (F- 4000) (from Zea mays) which has been compacted and granulated to produce 20-30 mesh granules (5000 gm), and 950 gm of magnesium stearate are added and the mixture is blended for 5 minutes. The mixture is then admixed with 10000 gm of enteric coated micro spheres containing 1000 gm of naloxone hydrochloride. This mixture makes 100,000 tablets each weighing 262 gm and containing 5 mg of oxycodone hydrochloride and 0.5 mg of naloxone hydrochloride. A tablet disintegrates in the U.S.P. disintegration test in less than 5 minutes. A tablet crushed and dispersed in 20 cc of water at 25° C. gives a thick gel which cannot be filtered through either cotton or coarse filter paper to obtain any filtrate, and cannot be drawn or discharged through an 18 gauge hypodermic needle.

Inasmuch as diversion of analgesics to parenteral abuse may be a theoretical possibility, in spite of the above safeguards, this diversion can be tracked by adding 10% by weight of a microtaggant such as Microtaggant^{tm}, which is available from Microtrace LLC, Minneapolis, MN 55449-7216. This material is a non-toxic additive that adds a identifying indicia to the product.

Optionally one may add 10000 gm of enteric coated microspheres containing 500 gm of naloxone hydrochloride prepared according to the procedure of Example 5 to prevent constipation as a side effect. The above mixture may be tabletted or filled into hard gelatin capsules.

### EXAMPLE 2

### (Methadone-Naloxone) (40 + 2gm)

A methadone-naloxone gum tablet was produced using the procedure described below:

### List of ingredients

16000 gm methadone hydrochloride U.S.P.
800gm naloxone hydrochloride
4000 gm starch U.S.P. (for paste)
10000 gm starch U.S. P. (for granulation)
160100 gm lactose U.S.P, anhydrous
3700 gm keltrol F (xanthan gum from Xanthamonas campestris)
14800 gm locust bean gum (from Seratonia siliqua)
2800 gm monobasic calcium phosphate, anhydrous
2800 gm dibasic calcium phosphate N.F., anhydrous
99200 gm microcrystalline cellulose
40000 gm Kelcoloid HVF 18-30 mesh (propylene glycol alginate)
2000 gm F D & C Yellow No. 5 lake
20000gm Zein F-4000, 20-30 mesh (from Zea mays)
3800 mg Magnesium stearate USP
(Optionally one can add 10000 gm of enteric coated microspheres containing 500 gm of naloxone hydrochloride prepared according to the procedure of Example 5 herein.

This composition is used to generate 400,000 tablets weighing 1.05 gm each.
Alternatively, the mixture may be filled into hard gelatin capsules in place of tabletting.

A starch paste is prepared by mixing 4000 gm of starch with 8000 gm of deionized water. A separate blend is prepared by mixing 2500 gm of starch, 16000gm of methadone hydrochloride and 40,000 gm of anhydrous lactose. The naloxone hydrochloride (800 gm) is dissolved in 1500 gm of deionized water. The starch, methadone hydrochloride, and anhydrous lactose. The wet granulation is passed through a No 10 mesh screen, spread on trays and dried for 18 hours at 120o F. The moisture content of the dried granulation is between 2.0-3.5%. The dried granulation is then consecutively passed through a No. 12 mesh screen and a No. 30 mesh screen.

To the dried granulation there are added 950 gm of xanthan gum (Keltrol F), 3700 gm of locust bean gum, 700 gm of monobasic calcium phosphate, 700 gm of dibasic calcium phosphate, 24,800 gm of microcrystalline cellulose (Avicel), and 500 gm of F .D. and C. yellow lake no. 5 and the mixture is blended for 15 minutes. Propylene glycol alginate (Kelcoloid HVF -10,000 gm), which has been compacted and granulated to produce 25-30 mesh granules 950 gm of magnesium stearate are added the mixture is blended for five minutes. Optionally, the mixture may then be admixed with 40,000 gm of enteric coated microspheres containing 2000 gm of enteric coated naloxone hydrochloride.

This mixture makes 400,000 tablets each weighing 1.05 gm and containing 40 mg of methadone hydrochloride and 2 mg of naloxone hydrochloride, in addition to 5 mg of enteric coated naloxone hydrochloride. Alternatively to tabletting the mixture may be filled into hard gelatin capsules.

### Example 3

### methadone-naloxone (5 + 0.25) For Analgesia

| Components | |
|---|---|
| Methadone hydrochoride | 500 gm |
| Naloxone hydrochloride | 25 gm |
| starch U.S.P. (for paste) | 4,000 gm |
| starch U.S.P. (for granulation) | 10,000 gm |
| lactose U.S.P. anhydrous | 160,100 gm |
| kehrol F | 3,700 gm |
| locust bean gum | 14,800 gm |
| monobasic calcium phosphate, anhydrous | 2,800 gm |
| di-calcium phosphate N.F.anhydrous | 2,800 gm |
| microcrystalline cellulose | 99,200 gm |
| Kelcoloid HVF | 40,000 gm |
| F D and C Yellow No. 5 lake | 2,000 gm |
| Zein F-4000 | 20,000 gm |
| Magnesium stearate USP | 3,800 gm |

The preparation of this dosage form is exactly as described for Example 1, except for the substitution of methadone hydrochloride for oxycodone hydrochloride.

### Example 4

### (Paregoric-Naloxone) Tablets or Capsules

| Components | |
|---|---|
| Opium powder | 400 gm |
| Naloxone hydrochloride | 20 gm |
| Starch U.S.P. (for paste) | 1000 gm |
| Starch U.S.P. (for granulation) | 2500 gm |
| Lactose (anhydrous) | 40000 gm |
| Keltrol F | 950 gm |
| Locust bean gum | 3700 gm |
| Monobasic calcium phosphate | 700 gm |
| Dibasic calcium phosphate | 700 gm |
| Microcrystalline cellulose | 24800 gm |
| Kelcoloid HVF 18 | 10000 gm |
| F .D. and C. yellow lake No.5 | 500 gm |
| Zein F-4000 | 5000 gm |
| Magnesium stearate U.S.P. | 950 gm |

Preparation is exactly as in example 1 substituting opium powder for oxycodone hydrochloride. This formula makes 100,000 tablets, or optionally, hard gelatin capsules wherein the dose of opium powder is 4mg and the dose of naloxone is 0.2mg and from one to two tablets may be taken every 4 hours up to six times a day for simple diarrhea.

### EXAMPLE 5

### Naloxone hydrochloride enteric coated non-pareil pellets

Naloxone hydrochloride antidiarrheal pellets, for inclusion into analgetic-naloxone tablets (see examples I-IV), having the following formulation were prepared.

| | |
|---|---|
| Naloxone hydrochloride | 0.134 kg |
| Sugar spheres (non-pareil) | 5.68 kg |
| Ethylcellulose, NF (Ethocel ) | 1.40 kg |
| Polysorbate 80 NF | 0.12 kg |
| Isopropyl alcohol USP* | 32.57 kg |
| (*Evaporated during processing) | |

Total weight 7.226 kg containing about 135,000 nonpareils, each containing about 1 mg of naloxone hydrochloride. Thus each final dosage forms should have about 3 to 10 beads.

Add the ethylcellulose to the isopropyl alcohol in a stainless steel tank. The naloxone hydrochloride (micronized) is added to the ethylcellulose solution with continuous agitation for at least 10 minutes with a homogenizer under conditions that avoid the formation of lumps or the introduction of air which causes foaming. The polysorbate 80 is then added while mixing in a homogenizer. The coating solution is sprayed onto the sugar spheres in a fluidized bed coater under the following conditions: product temperature 20-35° -C.; atomization pressure 2-4 bars; air volume 700-1800 m3/L. and a pump rate of 300-1500 mg/min. After spraying, the pellets are dried in the fluidized bed coater for approximately 10 minutes and then cooled and collected using a particle size separator.

The naloxone coated pellets are then coated with the enteric polymer to form enteric polymer membrane coated slow release pellets as follows:

| | |
|---|---|
| Naloxone coated pellets | 3.29 kg |
| Methacrylic acid copolymer (Eudragit S100) | 0.167 kg |
| Acetyl tributyl citrate | 0.027 kg |
| Talc USP | 0.056 kg |
| Isopropyl alcohol USP | 3.70 kg |
| Purified water USP | 0.10 kg |

The total weight of the coating solution plus pellets is 7.5 kg.

The acetyl tributyl citrate (plasticizer) is dissolved in the isopropyl alcohol in a stainless steel tank while homogenizing. The Eudragit S100 (poly methacrylate, (2-dimethyl aminoethyl) methacrylate, methyl methacrylate) 1:2:1) is added to the above mixture until it completely dissolves. Purified water is added to the polymer mixture to provide a clear solution. Then the talc is dispersed into the solution while mixing until a uniform coatingsuspension is formed. The suspension is continually stirred throughout the coating process to prevent sedimentation of the talc.
The following conditions are used during the spray coating: product temperature; first hour 35-40° C., thereafter 32-35°C; atomization pressure; 3-4 bar; pump rate; first hour 300-600 g/min; then 600-1500 gm/min.
After all coating suspension is consumed, dry the pellets in the fluidized bed for 5 minutes. Then cool the pellets until the temperature drops to 25-30° C. and discharge the pellets while dusting with talc. The pellets are then dried in an oven at 60 degrees C for at least 40 hours.

### EXAMPLE 6

### COATED NALOXONE PELLETS

### Preparation of naloxone pellets by the method of extrusion-spheronization

A mix of naloxone hydrochloride (60%) and Avicel PH101 (FMC, Belgium)(40%) is wetted with additional water (52.5%) in a planetary mixer. Wet powder masses are loaded into an Alexanderwerk GA65 gravity feed extruder.

The extrudate is spheronized in a Caleva 12 cm spheronizer fitted with a cross-hatch friction plate for 10 min. at 1250 rpm speed. After drying of the spheronized product at 45° C., sieving analysis is performed using a nest of standard sieves, and the desirable range of pellets was selected between 0.85 and 1.16 mm.

### Coat application by pan technology

A load of pellets (approximately 1 mm in diameter) is placed into a coating pan pre-roughened with polyvinylpyrrolidine/talc. A 20% w/v dispersion of guar-Eudragit S100 (1:4) in isopropanol-water (1:1) (350gms. of Eudragit S100; 1400gms of isopropanol; 100gms talc/3000gms of pellets (spheres)) is delivered to the cores and a stream of drying air at 60° C. was applied to the surface of the cores. Coat application is continued until a 40% coating weight gain was achieved. The microcapsules are cured at 450 C. for 12 hours in a forced air circulation oven, after which they are stored at 20° C. for 7-14 days prior to use. The coated sphere contain about 0.5 gm naloxone and thus the final dosage form will use about 6-20 beads.

### EXAMPLE 7

### (sustained release oxycodone plus naloxone for administration every 12 hours; the particular dose is dependent on the relative severity of the pain)

This analgetic preparation may be made in five sizes as follows:
Size A: 10 mg of oxycodone hydrochloride plus 0.5 mg of naloxone hydrochloride
Size B 20 mg of oxycodone hydrochloride plus 1.0 mg of naloxone hydrochloride
Size C 40 mg of oxycodone hydrochloride plus 2.0 mg of naloxone hydrochloride
Size D 80 mg of oxycodone hydrochloride plus 4.0 mg of naloxone hydrochloride
Size E 160 mg of oxycodone hydrochloride plus 8.0 mg of naloxone hydrochloride

In the sustained release mixture for each size of tablet or capsule one-third of the above content are in an immediate release form, one-third of the above content are compounded with 1/20 Eudragit L 100 for release in four hours in the jejunum, and one-third of the above content are compounded with 1/20 Eudragit S 100 for release in eight hours in the ileum. The preparation and coating of the sustained release pellets is carried out as described in Example 6.

| Components: | | |
|---|---|---|
| Oxycodone hydrochloride | For Size A | 1000 gm |
| | Size B | 2000 gm |
| | Size C | 4000 gm |
| | Size D | 8000 gm |
| | Size E | 16000 gm |
| Naloxone hydrochloride | For Size A | 50 gm |
| | Size B | 100 gm |
| | Size C | 200 gm |
| | Size D | 400 gm |
| | Size E | 800 gm |
| | | |
| Starch U.S.P. (for paste) | | 1000 gm |
| Starch U.S.P. (for granulation) | | 2500 gm |
| Lactose (anhydrous) | | 40000 gm |
| Keltrol F (xanthan gum from Xanthamonas) | | 950 gm |
| Locust bean gum (from Serotonia siliqua | | 3700 gm |
| Monobasic calcium phosphate | | 700 gm |
| Dibasic calcium phosphate | | 700 gm |
| Microcrystalline cellulose (Avicel) | | 24800 gm |
| Kelcoloid HVF 18 | | 10000 gm |
| F .D. and C. yellow lake No.5 | | 500 gm |
| Zein F 4000 20-30 mesh | | 5000 gm |
| Magnesium Stearate U.S.P. | | 950 gm |

### Example 8 (Sustained Release Oxycodone plus Naloxone)

Procedure carried out as in example 7, with the addition of 5 mg of naloxone hydrochloride per tablet to limit constipation.

## Claims

1. A solid pharmaceutical dosage form which comprises an opiate, an opiate antagonist and an amount of a hydrocolloid containing excipient which is effective to form a viscous, non-injectable matrix when said dosage form is contacted with water, which includes an amount of enteric coated opiate antagonist pellets which is effective to prevent opiate induced constipation.

2. A solid pharmaceutical dosage form as defined in claim 1 wherein the opiate is elected from the group consisting of morphine, codeine, dilaudid, pantopon, methadone, paregoric, pentazocine, buprenorphine, fentanyl, oxycodone, oxymorphone, hydromorphone, hydrocodone, propoxyphene, nalbuphine and meperidine.

3. A solid pharmaceutical dosage form as defined in claim 2 wherein the opiate is oxycodone.

4. A solid pharmaceutical dosage form as defined in claim 1 wherein the opiate antagonist is selected from the group consisting of naloxone, naltrexone, methylnaltrexone and naloxonazine.

5. A solid pharmaceutical dosage form as defined in claim 4 wherein the opiate antagonist is naloxone.

6. A solid pharmaceutical dosage form as defined in claim 1 wherein the hydrocolloid is selected from the group consisting of high viscosity hydroxypropyl methyl cellulose, agar, alginates, carrageenan, zein, guar gum, locust bean gum and xanthan gum.

7. A solid pharmaceutical dosage form as defined in claim 1 which also includes a diluent selected from the group consisting of microcrystalline cellulose and lactose.

8. A solid pharmaceutical dosage form as defined in claim 6 wherein the hydrocolloid is selected from the group consisting of locust bean gum, xanthan gum or mixtures thereof.

9. A solid pharmaceutical dosage form as defined in claim 1 which comprises oxycodone, naloxone powder, locust bean gum and xanthan gum.

10. A solid pharmaceutical dosage form as defined in claim 9 which includes an amount of naloxone in the form of enteric coated pellets which are effective to prevent the constipating effect of oxycodone.

11. A solid pharmaceutical dosage form as defined in claim 1 which comprises methadone, naloxone powder, locust bean gum and xanthan gum.

12. A solid pharmaceutical dosage form as defined in claim 11 which includes an amount of naloxone in the form of enteric coated pellets which are effective to prevent the constipating effect of methadone.

13. A solid pharmaceutical dosage form as defined in claim 1 which comprises opium powder, naloxone powder and locust bean gum.

14. A solid pharmaceutical dosage form as defined in claim 13 which includes an amount of naloxone in the form of enteric coated pellets which are effective to prevent the constipating effect of opium powder.

15. A solid pharmaceutical dosage form which comprises a controlled release dosage form of an opiate, an opiate antagonist and a hydrocolloid which is effective to form a viscous, non-injectable matrix when said dosage form is contacted with water, wherein said opiate, opiate antagonist and hydrocolloid are formulated into pellets (a); pellets (b) and pellets (c); pellets (a) comprise about one-third of said opiate, opiate antagonist and hydrocolloid in an immediate release form; pellets (b) comprise about one-third of said opiate, enteric coated opiate antagonist, and hydrocolloid in an a delayed release form which releases substantially all contents of the pellets in the jejunum; and pellets (c) comprise about one-third of said opiate, enteric coated opiate antagonist, and hydrocolloid in a delayed release form which releases substantially all of the contents of the pellets in the ileum.

16. A solid dosage form as defined in claim 15 wherein the opiate is oxycodone and the opiate antagonist is naloxone.

17. A method of preventing the formulation of a parenteral formulation of solid oral dosage form of an opiate, an opiate antagonist in immediate release form and an amount of enteric coated opiate antagonist pellets which is effective to prevent opiate induced constipation; said method comprising adding an amount of hydrocolloid to said solid oral dosage formulation of an opiate, so that when said solid oral dosage form contacts water, a matrix is formed which is too viscous to be injected via a hypodermic needle.

18. A solid pharmaceutical dosage form as defined in claim 1 wherein the hydrocolloid is selected from the group consisting of zein (from Zea mays); alginate (from locust bean gum (from Seratoriia siliqua), xanthan gum (from Xanthamonas campestris) or mixtures thereof.

## Patentansprüche

1. Feste pharmazeutische Dosierungsform, die ein Opiat, einen Opiatantagonisten und eine Menge eines Hydrokolloid enthaltenden Exzipiens, wirksam, um eine viskose, nicht-injizierbare Matrix zu bilden, wenn die Dosierungsform mit Wasser in Kontakt gebracht wird, umfasst, welche eine Menge an enterisch beschichteten Opiatantagonisten, Pellets, umfasst, die wirksam ist, um eine Opiat-induzierte Konstipation zu verhindern.

2. Feste pharmazeutische Dosierungsform, wie sie in Anspruch 1 definiert ist, wobei das Opiat ausgewählt ist aus der Gruppe, bestehend aus Morphin, Codein, Dilaudid, Pantopon, Methadon, Paregoric, Pentazocin, Buprenorphin, Fentanyl, Oxycodon, Oxymorphon, Hydromorphon, Hydrocodon, Propoxyphen, Nalbuphin und Meperidin.

3. Feste pharmazeutische Dosierungsform, wie sie in Anspruch 2 definiert ist, wobei das Opiat Oxycodon ist.

4. Feste pharmazeutische Dosierungsform, wie sie in Anspruch 1 definiert ist, wobei der Opiatantagonist ausgewählt ist aus der Gruppe, bestehend aus Naloxon, Naltrexon, Methylnaltrexon und Naloxonazin.

5. Feste pharmazeutische Dosierungsform, wie sie in Anspruch 4 definiert ist, wobei der Opiatantagonist Naloxon ist.

6. Feste pharmazeutische Dosierungsform, wie sie in Anspruch 1 definiert ist, wobei das Hydrokolloid ausgewählt ist aus der Gruppe, bestehend aus Hochviskositäts-Hydroxypropylmethylcellulose, Agar, Alginaten, Carragenan, Zein, Guargummi, Johannisbrotkerngummi und Xanthangummi.

7. Feste pharmazeutische Dosierungsform, wie sie in Anspruch 1 definiert ist, die auch ein Verdünnungsmittel, ausgewählt aus der Gruppe, bestehend aus mikrokristalliner Cellulose und Laktose, enthält.

8. Feste pharmazeutische Dosierungsform, wie sie in Anspruch 6 definiert ist, wobei das Hydrokolloid ausgewählt ist aus der Gruppe, bestehend aus Johannisbrotkerngummi, Xanthangummi und Gemischen davon.

9. Feste pharmazeutische Dosierungsform, wie sie in Anspruch 1 definiert ist, welche Oxycodon, Naloxonpulver, Johannisbrotkerngummi und Xanthangummi umfasst.

10. Feste pharmazeutische Dosierungsform, wie sie in Anspruch 9 definiert ist, welche eine Menge an Naloxon in der Form von enterisch beschichteten Pellets umfasst, welche wirksam sind, um die konstipierende Wirkung von Oxycodon zu verhindern.

11. Feste pharmazeutische Dosierungsform, wie sie in Anspruch 1 definiert ist, welche Methadon, Naloxonpulver, Johannisbrotkerngummi und Xanthangummi umfasst.

12. Feste pharmazeutische Dosierungsform, wie sie in Anspruch 11 definiert ist, welche eine Menge an Naloxon in Form von enterisch beschichteten Pellets umfasst, die wirksam sind, um die konstipierende Wirkung von Methadon zu verhindern.

13. Feste pharmazeutische Dosierungsform, wie sie in Anspruch 1 definiert ist, welche Opiumpulver, Naloxonpulver und Johannisbrotkerngummi umfasst.

14. Feste pharmazeutische Dosierungsform, wie sie in Anspruch 13 definiert ist, welche eine Menge an Naloxon in Form von enterisch beschichteten Pellets, welche wirksam sind, um die konstipierende Wirkung von Opiumpulver zu verhindern, umfasst.

15. Feste pharmazeutische Dosierungsform, umfassend eine Dosierungsform mit kontrollierter Freisetzung aus einem Opiat, einem Opiatantagonisten und einem Hydrokolloid, wirksam, um eine viskose, nicht-injizierbare Matrix zu bilden, wenn die Dosierungsform mit Wasser in Kontakt gebracht wird, wobei das Opiat, der Opiatantagonist und das Hydrokolloid zu Pellets (a); Pellets (b) und Pellets (c) formuliert sind; Pellets (a) umfassen etwa ein Drittel des Opiats, Opiatantagonisten und Hydrokolloids in einer Form mit unverzüglicher Freisetzung; Pellets (b) umfassen etwa ein Drittel des Opiats, enterisch beschichteten Opiatantagonisten und Hydrokolloids in einer Form mit verzögerter Freisetzung, welche im Wesentlichen alle Inhalte der Pellets in das Jejunum freisetzt, und Pellets (c) umfassen etwa ein Drittel des Opiats, enterisch beschichteten Opiatantagonisten und Hydrokolloids in einer Form mit verzögerter Freisetzung, die im Wesentlichen alle Inhalte der Pellets in das Ileum freisetzt.

16. Feste Dosierungsform, wie sie in Anspruch 15 definiert ist, wobei das Opiat Oxycodon ist und der Opiatantagonist Naloxon ist.

17. Verfahren zur Verhinderung der Formulierung einer parenteralen Formulierung einer festen oralen Dosierungsform eines Opiats, Opiatantagonisten in einer Form mit unmittelbarer Freisetzung und einer Menge an enterisch beschichteten Opiatantagonisten Pellets, die wirksam ist, um eine Opiat-induzierte Konstipation zu verhindern, wobei das Verfahren umfasst: Zugeben einer Menge an Hydrokolloid zur festen oralen Dosierungsformulierung eines Opiats, so dass, wenn die feste orale Dosierungsform mit Wasser in Kontakt kommt, eine Matrix gebildet wird, die zu viskos ist, um über eine hypoderme Nadel injiziert zu werden.

18. Feste pharmazeutische Dosierungsform, wie sie in Anspruch 1 definiert ist, wobei das Hydrokolloid ausgewählt ist aus der Gruppe, bestehend aus Zein (aus Zea mays); Alginat (aus Johannisbrotkerngummi (aus Seratoriia siliqua)), Xanthangummi (aus Xanthamonas campestris) oder Gemischen davon.

## Revendications

1. Forme galénique solide qui comprend un opiacé, un antagoniste d'opiacé et une quantité d'un excipient contenant un hydrocolloïde qui est efficace pour former une matrice visqueuse, non injectable lorsque ladite forme galénique est mise en contact avec de l'eau, qui comprend une quantité de granules à enrobage entérosoluble d'antagoniste d'opiacé qui est efficace pour prévenir la constipation induite par l'opiacé.

2. Forme galénique solide selon la revendication 1, dans laquelle l'opiacé est choisi dans le groupe constitué par la morphine, la codéine, le dilaudide, le pantopon, la méthadone, l'élixir parégorique, la pentazocine, la buprénorphine, le fentanyl, l'oxycodone, l'oxymorphone, l'hydromorphone, l'hydrocodone, le propoxyphène, la nalbuphine et la mépéridine.

3. Forme galénique solide selon la revendication 2, dans laquelle l'opiacé est l'oxycodone.

4. Forme galénique solide selon la revendication 1, dans laquelle l'antagoniste d'opiacé est choisi dans le groupe constitué par la naloxone, la naltrexone, la méthylnaltrexone et la naloxonazine.

5. Forme galénique solide selon la revendication 4, dans laquelle l'antagoniste d'opiacé est la naloxone.

6. Forme galénique solide selon la revendication 1, dans laquelle l'hydrocolloïde est choisi dans le groupe constitué par l'hydroxypropylméthylcellulose à viscosité élevée, l'agar-agar, les alginates, la carraghénine, la zéine, la gomme de guar, la gomme de caroube et la gomme de xanthane.

7. Forme galénique solide selon la revendication 1, qui comprend également un diluant choisi dans le groupe constitué par la cellulose microcristalline et le lactose.

8. Forme galénique solide selon la revendication 6, dans laquelle l'hydrocolloïde est choisi dans le groupe constitué par la gomme de caroube, la gomme de xanthane ou leurs mélanges.

9. Forme galénique solide selon la revendication 1, qui comprend l'oxycodone, la poudre de naloxone, la gomme de caroube et la gomme de xanthane.

10. Forme galénique solide selon la revendication 9, qui comprend une quantité de naloxone sous la forme de granules à enrobage entérosoluble qui sont efficaces pour prévenir l'effet constipant de l'oxycodone.

11. Forme galénique solide selon la revendication 1, qui comprend la méthadone, la poudre de naloxone, la gomme de caroube et la gomme de xanthane.

12. Forme galénique solide selon la revendication 11, qui comprend une quantité de naloxone sous la forme de granules à enrobage entérosoluble qui sont efficaces pour prévenir l'effet constipant de la méthadone.

13. Forme galénique solide selon la revendication 1, qui comprend la poudre d'opium, la poudre de naloxone et la gomme de caroube.

14. Forme galénique solide selon la revendication 13, qui comprend une quantité de naloxone sous la forme de granules à enrobage entérosoluble qui sont efficaces pour prévenir l'effet constipant de la poudre d'opium.

15. Forme galénique solide qui comprend une forme de dosage à libération prolongée d'un opiacé, d'un antagoniste d'opiacé et d'un hydrocolloïde, qui est efficace pour former une matrice visqueuse, non injectable lorsque ladite forme galénique est en contact avec de l'eau, dans laquelle lesdits opiacé, antagoniste d'opiacé et hydrocolloïde sont formulés en granules (a), granules (b) et granules (c); les granules (a) comprennent environ un tiers desdits opiacé, antagoniste d'opiacé et hydrocolloïde sous une forme à libération immédiate; les granules (b) comprennent environ un tiers desdits opiacé, antagoniste d'opiacé à enrobage entérosoluble et hydrocolloïde sous une forme à libération retardée qui libère sensiblement tous les contenus des granules dans le jéjunum; et les granules (c) comprennent environ un tiers desdits opiacé, antagoniste d'opiacé à enrobage entérosoluble et hydrocolloïde sous une forme à libération retardée qui libère essentiellement tous le contenu des granules dans l'iléon.

16. Forme galénique solide selon la revendication 15, dans laquelle l'opiacé est l'oxycodone et l'antagoniste d'opiacé est la naloxone.

17. Procédé de prévention de la formulation d'une formulation parentérale de forme galénique orale solide d'un opiacé, un antagoniste d'opiacé sous une forme à libération immédiate et une quantité de granules à enrobage entérosoluble d'antagoniste d'opiacé qui est efficace pour prévenir la constipation induite par l'opiacé, ledit procédé comprenant l'étape consistant à ajouter une quantité de hydrocolloïde à ladite formulation galénique orale solide d'un opiacé de sorte que lorsque ladite forme galénique orale solide est en contact avec de l'eau, une matrice est formée qui est trop visqueuse pour être injectée par aiguille hypodermique.

18. Forme galénique solide selon la revendication 1, dans laquelle l'hydrocolloïde est choisi dans le groupe constitué par la zéine (provenant de *Zea mays*), l'alginate (provenant de la gomme de caroube (provenant de *Seratoriia siliqua*), la gomme de xanthane (provenant de *Xanthamonas campestris*) ou leurs mélanges.
